# EUROPEAN PATENT SPECIFICATION

(11) **EP 2 699 905 B1**
(45) Date of publication and mention of the grant of the patent: **14.04.2021**
(21) Application number: 12721332.0
(22) Date of filing: 17.04.2012
(51) Int. Cl.: G01N 33/53, G01N 33/68

(54) **Immunoassay for quantifying multiple antigen-specific immunoglobulins**
Immunoassay zur Quantifizierung mehrerer Antigen-spezifischer Immunglobuline
Immunoessai pour quantifier plusieurs immunoglobulines spécifiques d'antigène

(30) Priority: 18.04.2011 GB 201106478
(43) Date of publication of application: 26.02.2014
(73) Proprietor: LumiraDx UK Limited, London SE1 2AQ (GB)
(72) Inventor: CRISANTI, Andrea, London SW5 9DB (GB); MACCARI, Mauro, London SW6 1SL (GB); BALDRACCHINI, Francesca, London N1 2LW (GB)
(74) Representative: Stratagem IPM Limited
(86) International application number: PCT/GB2012/050846
(87) International publication number: WO 2012/143709

(56) References cited:
- WO-A2-2011/101670
- HARWANEGG CHRISTIAN ET AL: "Protein microarrays for the diagnosis of allergic diseases: state-of-the-art and future development", CLINICAL CHEMISTRY AND LABORATORY MEDICINE, WALTER DE GRUYTER & CO, BERLIN, NEW YORK, vol. 43, no. 12, 1 December 2005 (2005-12-01), pages 1321-1326, XP008137085, ISSN: 1434-6621, DOI: 10.1515/CCLM.2005.226 [retrieved on 2005-11-28]
- DEINHOFER K ET AL: "Microarrayed allergens for IgE profiling", METHODS : A COMPANION TO METHODS IN ENZYMOLOGY, ACADEMIC PRESS INC., NEW YORK, NY, US, vol. 32, no. 3, 1 March 2004 (2004-03-01), pages 249-254, XP004488975, ISSN: 1046-2023, DOI: 10.1016/J.YMETH.2003.08.018 cited in the application
- DERER M M ET AL: "APPLICATION OF THE DOT IMMUNOBINDING ASSAY TO ALLERGY DIAGNOSIS", JOURNAL OF ALLERGY AND CLINICAL IMMUNOLOGY, ELSEVIER, AMSTERDAM, NL, vol. 74, no. 1, 1 July 1984 (1984-07-01), pages 85-92, XP000562613, ISSN: 0091-6749, DOI: 10.1016/0091-6749(84)90093-9

## Description

The invention relates to an immunoassay method of quantifying IgE levels in a sample, methods of calibrating a device suitable for carrying out such quantification and a system that enables such quantification.

Immunoassays are methods that utilise the binding capacity of antibodies. Often, immunoassays are used to assay for the presence of a particular antigen-specific antibody in a sample. This is done by washing the sample over the particular antigen immobilised on a solid support, and subsequently visualising any bound antibody using various techniques.

Generally, immunoassays require the use of calibrators to assign values or concentrations to unknown samples. In a classical immunoassay, a set of calibrators is run, a calibration curve of signal versus concentration is plotted and the concentration of the unknown samples determined by interpolation.

Allergic conditions are characterised by inappropriate and exaggerated immune responses to innocuous environmental antigens. These antigens are collectively called allergens. Immune responses to allergens include a first phase of sensitization consisting of (i) processing of the allergens by antigen presenting cells (APCs), (ii) presentation of the processed allergens by the APCs to T helper 0 (Th0) naive cells, (iii) differentiation of the Th0 naïve cells to Th2 cells, and (iv) stimulation of B cells by the Th2 cells, leading to the production and secretion of allergen-specific IgE by the B cells.

Each specific allergen will stimulate the production of an IgE specific to that allergen. IgE antibodies can interact with two different cell types; mast cells and basophils, which contain histamine-containing granules.

When the same allergen that has elicited the sensitization phase enters the body a second time and is recognised by the appropriate mast-cells and basophils, it stimulates a second phase of the allergy mechanism known as the "challenge phase". The allergen binds to its specific IgE presented on the surface of mast-cells and basophils triggering a mechanism which eventually leads to degranulation of the mast cells and basophils and secretion of histamine, which is responsible for the inflammatory reaction typical of an allergic reaction. The severity of the ensuing allergic reaction corresponds with the level of allergen-specific IgE in that individual. Therefore, it is important to detect and quantify the concentration of IgEs raised against particular allergens in an individual to enable identification of allergic (or atopic) individuals and characterisation of their allergies.

Quantitative immunoassays for the diagnosis of allergy normally use or refer to the World Health Organization (WHO) International Reference Preparation 75/502 to build their calibration systems. This is a freeze-dried human serum sample with an assigned IgE reactivity (Kontis K, et al (2006) Correlation of the Turbo-MP RIA with ImmunoCAP FEIA for Determination of Food Allergen-Specific Immunoglobulin-E. Ann Clin Lab Sci. 36(1): 79-87; Bousquet J et al (1990) Comparison between RAST and Pharmacia CAP system: A new automated specific IgE assay. J Allergy Clin Immunol. 85(6):1039-43; Reference for the product: http://www.nibsc.ac.uk/documents/ifu/75-502.pdf).

Measured response values for allergen-specific IgE antibodies are typically evaluated against a total IgE calibration curve (WHO International Reference Preparation) and expressed as concentration of Allergen specific Units per litre (kUA/I). The IgE reference curve is used to describe the dose-response curve for all the allergens tested. This requires that the concentrations of allergenic components that are immobilised on a solid support for the immunoassay are optimised such that the dose-response curves for the IgE and the allergens show the same trend. To optimise the concentrations of the allergenic concentrations for the dose response curves, different concentrations of the allergens are tested against a panel of samples at known reactivity to identify the concentration that gives a dose-response curve similar in shape and slope to the one generated using the WHO International Reference total IgE curve.

Immunoassays presently used for the diagnosis of allergic disease include: Radioallergosorbent Test (RAST), Enzyme-Linked Immunosorbent Assay (ELISA), and ImmunoCAP.

RAST involves covalently coupling an allergen (or other antigen) to a paper disk solid phase. The paper disk is then incubated with serum from a patient whose allergenic status is to be investigated. If antibodies against the allergen are present in the serum, they react with the conjugated allergen and binding is revealed with radio-labelled anti-IgE antibodies. In its original form, the results of the test were reported in classes or arbitrary units by interpolating from a heterologous IgE anti-birch pollen reference curve. Birch allergen coupled to the paper disk is incubated with known reactivity serum samples and the reference curve is generated by plotting the signal obtained against the known IgE concentration. WHO/NIBSC International Reference IgE Preparation, as above, is generally used for calibration of the birch reference system.

Similarly, ELISA involves allergens (or other antigens) adsorbed to a solid phase (typically a plastic multi-welled plate) incubated with serum of a patient to be investigated. Binding of IgE in the sample to the adsorbed allergens is revealed by incubating the IgE bound to the allergens adsorbed onto the solid phase with an enzyme-linked anti-lgE antibody and then adding an appropriate substrate for the enzyme. Catalysis of the substrate leads to a colour change on the plate. Measurement of the colour intensity allows quantification of the serum IgE by interpolation of the colour signal to a reference curve. The reference curve is generated by coating ELISA wells with capture anti-human IgE antibodies and incubating them first with WHO/NIBSC International Reference IgE Preparation standard, followed by the enzyme-linked anti-IgE antibodies with an appropriate substrate. The concentration of the capture anti-lgE remains constant across the reference wells and the reference IgE preparation is titrated to obtain the reference curves.

As an example ELISA RV-5 kit produced by ALLERGOPHARMA consists of a single concentration of allergens adsorbed to papers disks placed on the bottom of flat 96-well plates along with disks coated with a single concentration of capture anti-human IgE. While allergen-coated disks are incubated with patient serum, capture anti-lgE-coated disks are hybridized with human IgE Preparation derived from the WHO/NIBSC International Reference. The reference curve is then generated by plotting the signal intensity obtained from the capture anti-lgE-coated wells against the known WHO/NIBSC IgE concentrations. The fully automated Enzyme Immunoassay (EIA) utilized by HYCOR for Allergy testing is based on the same principle.

The CAP system-PHADIA (reference method) essentially differs from the above methods in the nature of the solid phase - ImmunoCAP. The solid phase of ImmunoCAP is a CNBr-activated cellulose derivative which has higher binding capacity compared to other substrates (David W. (2006), The immunoassay handbook, published by Elsevier Ltd). Allergens of interest are covalently coupled to a hydrophilic carrier polymer encased within a capsule. The carrier consists of the cellulose derivative with high protein binding properties. The ImmunoCAP can react with specific IgE in patient serum and after washing away the unbound IgE, enzyme-labelled anti-lgE antibodies are added to form a complex which is then incubated with a fluorogenic substrate. As with the ELISA method, the colour intensity provides an indication of the level of allergen-specific IgEs in the serum by interpolation to a heterologous total serum-lgE dose-response curve used for calibration. The assay is calibrated against the WHO standard for IgE and includes two sets of calibrators: 0.35-100 kUA/I (for specific IgE Ab and low range total IgE) and 2-2000 kUA/I (for wide range total IgE). The anti-lgE is designed to permit a wider measuring range with the same initial slope of the dose response curve. All solid-phase allergens are then individually optimized for maximum capacity and response and low background noise relative to the measuring range.

The ImmunoCAP ISAC Kit produced by PHADIA is a microarray-based test for the diagnosis of allergy. This is based on modern biochip technology. ImmunoCAP ISAC is a miniaturized immunoassay platform that allows for multiplex measurement of specific IgE antibodies to many allergen components. Purified natural or recombinant allergen (40 common allergen sources) components are immobilized on a solid support (biochip). This is a two step assay. First, IgE antibodies from patient serum bind to the immobilized allergen components. Second, after a short washing step, allergen-bound IgE antibodies are detected by a fluorescence-labelled anti-lgE antibody. ImmunoCAP ISAC is a semiquantitative test and results are reported in ISAC Standardized Units (ISU).

Deinhofer et al (2004) Methods: 32: 249-254 describes the application of microarray technology to multi-allergen test systems.

EP 1 322 960 B1 describes a microarray-based allergen test system.

WO2011/101670 by the present applicants interpolates average signals of streptavidin captured biotin against a replicate generated external reference curve.

Harwanegg et al (Clin. Chem. And Lab. Medicine, Dec 2005, vol 43(12), 1321-1326) describes microarrays for the diagnosis of allergic diseases wherein the IgE antibody is immobilised, not the anti-lgE antibodies. Further, this does not teach the use of a binding capacity curve as disclosed herein.

The listing or discussion of an apparently prior-published document in this specification should not necessarily be taken as an acknowledgement that the document is part of the state of the art or is common general knowledge.

The invention seeks to address problems with the above immunoassays. The invention provides a more accurate immunoassay for quantifying IgE levels in test samples.

In a first aspect the invention provides a method of quantifying multiple antigen-specific immunoglobulins in a test sample, the method comprising the steps of;
(i) assaying binding of a series of samples, for example serum samples, containing immunoglobulin of known antigen reactivity, the immunoglobulin being of the same subtype as that in the test sample, to multiple recombinant or purified antigen components or fragments thereof immobilised on a first solid support,
(ii) comparing the level of binding in step (i) with the known reactivity to produce a dose response curve for each antigen component or fragment thereof,
(iii) assaying binding of a serial dilution of a reference immunoglobulin sample of the same immunoglobulin subtype as that used in part (i) with a known total amount of immunoglobulin to a serial dilution of anti-immunoglobulin antibodies, fragments or derivatives thereof, immobilised on the first, or a second, solid support,
(iv) comparing the level of binding in step (iii) with the known total amount of reference immunoglobulin to produce a binding capacity curve for each anti-immunoglobulin antibody, fragment or derivative dilution,
(v) comparing the dose response curves produced in step (ii) with the binding capacity curves produced in step (iv), identifying the binding capacity curve that most closely matches the dose-response curve for each antigen or fragment thereof, and assigning the identified binding capacity curve as a binding capacity curve to each antigen or fragment thereof,
(vi) assaying binding of antigen-specific immunoglobulin in the test sample to the recombinant or purified antigen components or fragments thereof immobilised on the first, second, or a third solid support, and
(vii) comparing the level of binding in step (vi), with respect to each individual antigen or fragment thereof, to the binding capacity curve assigned to that antigen or fragment thereof in step (v) and quantifying the level of antigen-specific immunoglobulin present in the test sample.

The samples containing known immunoglobulin reactivity may be any sample containing known reactivity of immunoglobulin to the specific antigens. It is preferred that the samples contain known IgE reactivity. The reactivity of the sample will have been determined prior to their use in the methods of the present invention through the use of an appropriate immunoassay, as would be appreciated by a skilled person. Examples of appropriate immunoassays are provided above, for example ELISA. It is preferred that the samples are serum samples, for example human serum samples. The reactivity may be expressed in International Units per millilitre (IU/ml). Dose-response curves are produced, for example, by plotting fluorophore signal intensity obtained in an immunoassay against IgE reactivity expressed in International Unit/ml.

It is intended that the series of samples used in step (i) comprise a panel of samples; each sample may be reactive with one or more of the immobilised, or other, antigens. This step utilises the differing properties of different samples, which samples can each have different levels of reactivity towards the same antigens to provide a wide range of different binding levels to each antigen. For example, a Sample 1 may have reactivity x for Antigen A, a Sample 2 may have reactivity y for Antigen A, and a Sample 3 may have reactivity z for Antigen A. When the reactivity of Samples 1 to 3 to Antigen A are plotted on a curve, a dose response curve is generated. Thus, these samples with differing reactivity to the same antigens, and to different antigens, are used to build a dose-response curve for each immobilised antigen to be used in later steps on the method.

It is envisaged that the known reactivity sample and/or the test sample are samples obtained from a patient, for example a human. The sample may be a serum sample, whole blood sample, plasma sample, lymph sample, cerebrospinal fluid sample, bone marrow sample, lung aspirate sample, urine sample, stool sample, saliva sample, sputum sample, tissue sample or any other sample that may contain immunoglobulin. It is preferred that the samples are serum samples containing known IgE reactivity.

The reference immunoglobulin sample contains an appropriate class of immunoglobulins according to the class of immunoglobulins that are intended to be detected in the test sample. For example, if the immunoassay is for the detection of IgE in the test sample, then the reference immunoglobulin sample will contain known total IgE. The reference immunoglobulin sample may be any sample of immunoglobulin whose total immunoglobulin concentration is known. For example, when the immunoglobulin is IgE the reference IgE sample may be the WHO/NIBSC International Reference. The total IgE may be expressed in International Unit/ml.

Alternative arrangements are envisaged where the immunoglobulin is IgG, IgA, and/or IgM, or any other immunoglobulin subclass that may be used in the methods of the invention. Appropriate anti-immunoglobulins would be provided for generating binding capacity curves to represent specific antigen binding capacity with each of these different classes of immunoglobulin. In other words, when the immunoglobulin subclass to be detected is IgA, the reference immunoglobulin and known reactivity immunoglobulin sample would contain appropriate IgA and the anti-immunoglobulin antibody would be anti-lgA.

The anti-immunoglobulin antibodies provided in step (iii) of the first aspect immobilised on the first, or a further, solid support are directed to the antibody class that is intended to be detected in the test sample. For example, if allergen-specific IgE antibodies are intended to be detected in the test sample, then the serum samples and the reference sample will contain IgE of known reactivity and known total IgE respectively. Thus, the anti-immunoglobulin antibodies will be anti-lgE antibodies. The antibody subclass of the anti-immunoglobulin antibodies would generally be IgG. Thus, it is envisaged that the anti-immunoglobulin antibodies may be anti-lgE, IgG antibodies.

By "antigen" we include the meaning of any compound that contains an epitope that is specifically recognised by an immunoglobulin. Thus, the antigen may be derived from natural extracts, it may be a recombinant protein, or another protein or other molecule (such as a polysaccharide) purified from natural extracts, or any other source. It is preferred that the antigen is an allergen, i.e. an antigen that is recognised as being capable of causing an allergic reaction in an individual upon contact with that individual. The antigen may be a characterised allergen, or a yet to be characterised allergen. It is envisaged that the antigen may be any compound that is specifically recognised by IgE molecules.

Examples of allergen components that may be included on the solid support include: Der p1 and Der p2 - major allergenic molecules in the Dust Mite, *Dermatophagoides pteronyssinus*; Bet v1 and Bet v2 - major allergenic molecules of Birch pollen; Phl p1, Phl p5, Phl p2 and Phl p6 - major allergenic molecules of Timothy Grass pollen.

Step (ii) of the first aspect above provides a dose response curve for each antigen contained on the solid support. The antigen concentrations on the solid support may be optimised such that an appropriate response is obtained. Antigens (for example allergens) may be optimized in terms of concentration of protein and by way of the most appropriate buffer. Optimisation of the antigen concentration and buffer is performed before the methods of the present invention are carried out. Optimisation is carried out by identifying, for each antigen, the most appropriate concentration of protein and most appropriate buffer in which to dilute the protein that gives the highest concordance in terms of reactivity when compared with samples at known reactivity for that given antigen. Examples of appropriate buffers for use in solubilising the antigens to be immobilised on the solid support and optimising the antigen concentration include: Phosphate buffer saline pH 7.4; Phosphate buffer saline pH 7.4 with 0.1 g/l Tween® 20; and/or Phosphate buffer saline pH 7.4 with 10 % Glycerol.

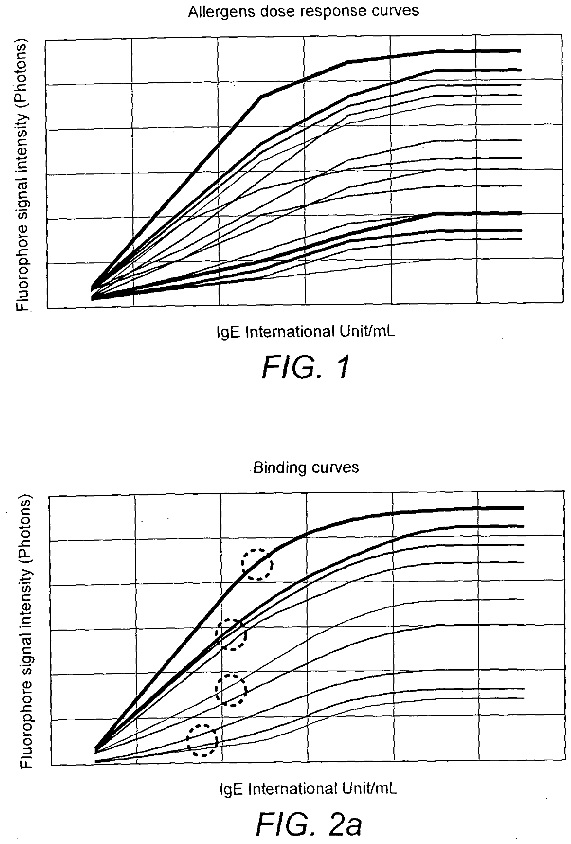

Following completion of steps (i) to (iv) of the first aspect, the skilled person will be in possession of a dose response curve for each immobilised antigen and a binding capacity curve for each concentration of anti-immunoglobulin antibody present on the first, or the further solid support. Thus, two graphs will be produced: the first comparing binding intensity for each antigen with the reactivity of antigen-specific immunoglobulin present in a known sample (see Figure 1 for an example of such a curve with IgE containing samples); the second comparing binding intensity for each dilution of anti-immunoglobulin antibody with increasing concentrations of total immunoglobulin present in a reference sample (see Figure 2A for an example of such a curve with IgE containing samples).

Step (v) of the first aspect provides for a comparison of these two graphs to match the curves produced for each antigen with the curves produced for each anti-immunoglobulin antibody concentration. Such comparison may be carried out visually or by some other means, such as with the use of computer software.

Once each antigen sample is matched with a particular anti-immunoglobulin concentration that immunoglobulin concentration is assigned to that antigen and is later used as a more accurate reference, or calibration, curve for that antigen, which more accurately describes that particular antigen's binding capacity. Thus, the concentration of antibodies specific to that antigen in a test sample may be more accurately elucidated through the use of the newly assigned binding capacity calibration curve for that antigen.

The inventors have identified that the use of a single reference curve for calibrating immunoassays for multiple antigens, particularly multiple allergens, as is standard practice in the art, is inadequate to describe the different binding capacities that different allergens exhibit. The inventors found that when immunoassays are performed on serum samples with known IgE reactivity, incubated with different allergen extracts, different dose-response curves are obtained with the data generated for each allergen. This is exemplified in Figure 1. Thus, in the example of allergen testing, the calibration systems used in previous allergen immunoassays were inadequate for providing accurate quantitative information for IgE reactivity of serum samples when multiple allergens are tested in a multiplex assay.

The inventors sought to provide a calibration system that would address the disadvantages of the available immunoassays and provide a more accurate quantitative immunoassay. The step of assigning a different reference curve to each antigen as in the present invention, based on their binding capacity, enables more accurate quantification of specific immunoglobulin in a test sample. In the example of allergen testing, the present invention describes allergen dose-response behaviour in a more accurate way than previous assays.

The binding capacity curves may be loaded into software that controls immunoassay analysing instruments to be used as standards for future assays. Internal controls on each assay may be used to compensate for minor environmental variations in each assay. Nevertheless, it is envisaged that when new batches of allergen are prepared for loading onto a solid support, the binding capacity curves may be adjusted appropriately or new binding capacity curves produced, as taught herein, to ensure accuracy of the assay. Such quality control activities will be readily understood by the skilled person.

Steps (vi) and (vii) of the first aspect utilise the binding capacity curves generated in the earlier steps to quantify antigen-specific immunoglobulin levels in the test sample. It is envisaged that all of the immobilised components described in the first aspect may be immobilised on the same, or different solid supports, as required by the assay equipment that is utilised to carry out the method. Thus, all the appropriate immobilised components may be immobilised on the same solid support, for example chip, including antigens, capture immunoglobulins and positive and negative controls. Nevertheless, it is envisaged that each sample will be incubated with a different solid support, for example chip, to prevent cross-contamination. Thus, a number of solid supports, for example chips, will be used in each assay. For example, if 50 serum samples are to be incubated on the solid support, 50 separate solid supports with the appropriate immobilised antigens/immunoglobulins/controls may be used. Equally, each reference sample may be incubated on a different solid support to prevent cross contamination. The assay equipment utilised will influence the exact mechanics of incubation of the samples, as would be understood by a person of skill in the art.

In a preferred embodiment, the reference immunoglobulin sample (for example the WHO international standard IgE) is used at final immunoglobulin concentrations ranging from 0.1 to 100 IU/ml. Preferably, the anti-immunoglobulin antibody to be immobilised on the solid support (for example anti-lgE antibody) is used at concentrations ranging from 30 to 0.1 µg/ml.

The term "immunoglobulin(s)" is used herein interchangeably with the term "antibody" or "antibodies".

The first aspect may include a further step wherein step (iv) further comprises clustering the binding capacity curves produced in step (iv) into representative binding capacity curves to represent different levels of binding capacity, for example, very high binding, high binding, medium binding and low binding, and the representative binding curves are used as the binding capacity curves produced in step (iv)

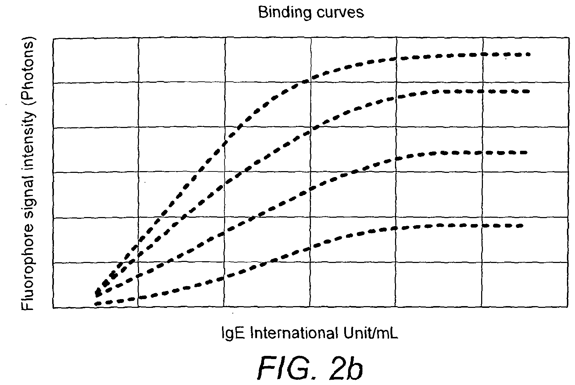

It is envisaged that including this further step of providing fewer consolidated binding capacity curves may simplify data management, thus simplifying the calibration process. These binding capacity curves, as exemplified in Figure 2B may be stored in the software of immunoassay analyser instruments for future analysis of samples.

By "anti-immunoglobulin antibodies, fragments or derivatives thereof" we include the meaning that the antibodies comprise an antibody or antigen binding fragment thereof such a Fab-like molecules; Fv molecules; single-chain Fv (ScFv) molecules where the V_{H} and V_{L} partner domains are linked via a flexible oligopeptide and single domain antibodies (dAbs) comprising isolated V domains, but it may also be any other ligand which exhibits the preferential binding characteristic mentioned above.

In a second aspect, the invention provides a method of calibrating a device suitable for assaying binding of multiple antigen-specific immunoglobulins to multiple antigens or fragments thereof immobilised on a solid support, the method comprising the steps of;
(i) assaying binding of a series of samples, for example serum samples, containing immunoglobulin of known antigen reactivity to multiple recombinant or purified antigen components or fragments thereof immobilised on a first solid support,
(ii) comparing the level of binding in step (i) with the known reactivity to produce a dose response curve for each antigen component or fragment thereof,
(iii) assaying binding of a serial dilution of a reference immunoglobulin sample of the same subtype as that used in part (i) with a known total amount of immunoglobulin to a serial dilution of anti-immunoglobulin antibodies, fragments or derivatives thereof, immobilised on the first, or a second, solid support,
(iv) comparing the level of binding in step (iii) with the known total amount of reference immunoglobulin to produce a binding capacity curve for each anti-immunoglobulin antibody, fragment or derivative dilution,
(v) comparing the dose response curves produced in step (ii) with the binding capacity curves produced in step (iv), identifying the binding capacity curve that most closely matches the dose-response curve for each antigen or fragment thereof, and assigning the identified binding capacity curve as a binding capacity curve to each antigen or fragment thereof, and
(vi) inputting the binding capacity curves generated in step (v) into the device such that the binding capacity curves for each antigen can be interpolated with signals produced from samples containing unknown amounts of immunoglobulin that specifically binds that antigen.

The second aspect may include a further step wherein step (iv) further comprises clustering the binding capacity curves produced in step (iv) into representative binding capacity curves to represent different levels of binding capacity, for example, very high binding, high binding, medium binding and low binding, and the representative binding capacity curves are used as the binding capacity curves produced in step (iv).

Once such a device has been calibrated using the method of the second aspect, it may be utilised to assay and quantify antigen-specific immunoglobulin in test samples.

For example, a microarray slide (solid support) following the appropriate treatment to visualise the antigens bound to antibody can be read using an ADAM instrument (Microtest Matrices Ltd). Raw data are collected and used to calculate the dose-response curve. The output of the instrument is a textual file where all the dots of the microarray are listed; they are described by the coordinates and a numeric value that is the photons count emitted by each dot on the microarray. Using a numerical computing environment similar to Matlab, all the data obtained from the reader are computed and a set of factors that describe the dose-response curves are generated. The ADAM instrument uses these factors to build the internal Master Calibration Curve, inserted in a configuration file.

In preferred embodiments of the first and second aspects, the antigens are allergens, the immunoglobulin is IgE and the anti-immunoglobulin antibodies are anti-lgE antibodies. Thus, in such embodiments, the methods may be used in the detection of allergies in patients to certain allergens by assaying samples obtained from the patient for IgE reactivity to the allergens components or fragments thereof immobilised on the solid support. Such information may aid in the diagnosis of allergy to particular allergens.

Examples of allergens that may be immobilised on the solid support include those listed in Table 1.

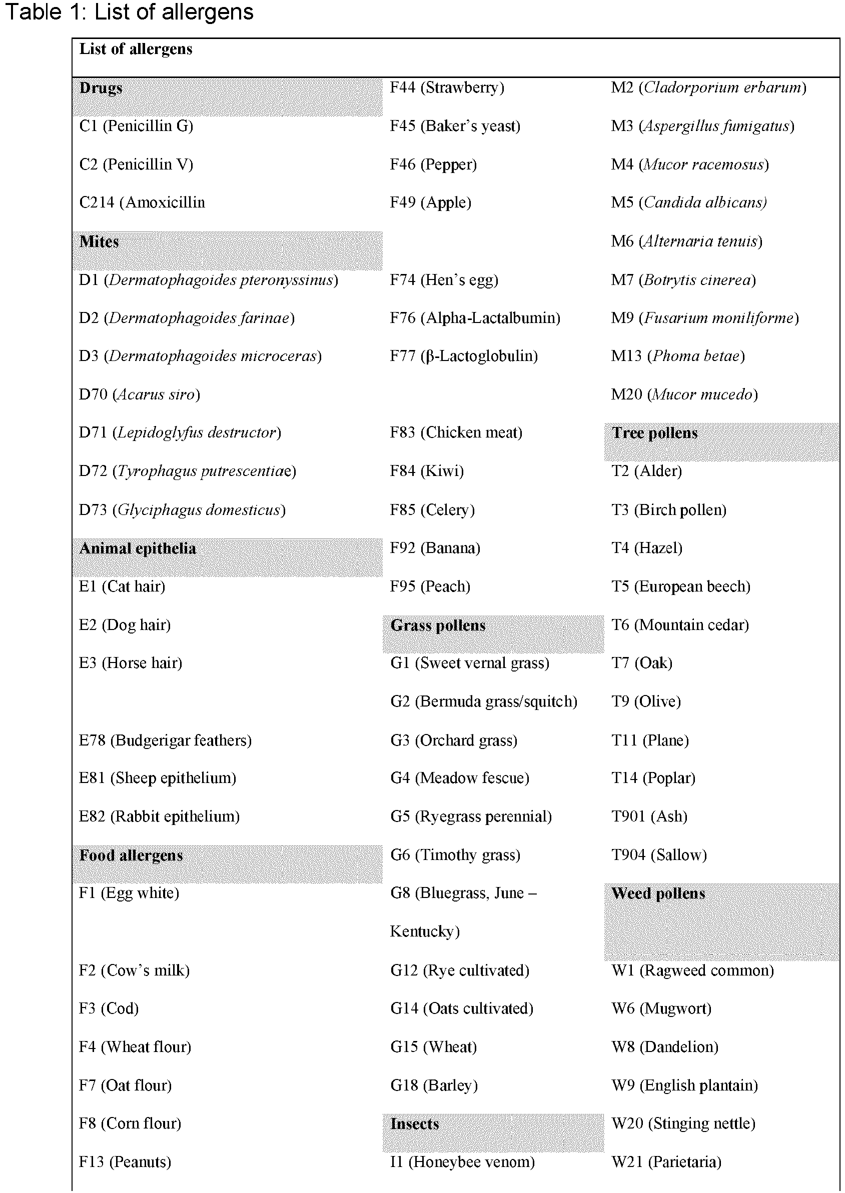

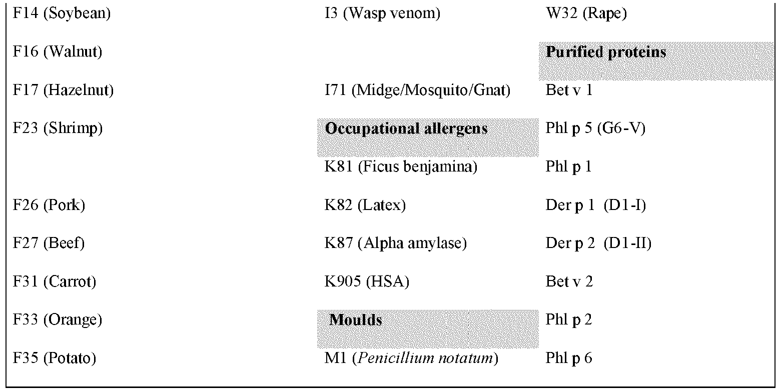

In a third aspect, there is provided a multi-allergen test system comprising a serial dilution of anti-lgE antibodies, fragments or derivatives thereof immobilised on a first solid support. Such multi-allergen test system may be used in the methods of the earlier aspects of the invention.

The system further comprises allergen components or fragments thereof, preferably recombinant or purified allergen components or fragments thereof immobilised on the first, or a second, solid support.

The system further comprises positive and negative controls immobilised on a first, second or third solid support.

In a fourth aspect, the invention provides for the use of a kit of parts comprising the multi-allergen test systems of the third aspect, and one or more of the following:
i) a reference IgE sample;
ii) a first antibody preparation comprising first antibodies that bind IgE;
iii) a second antibody preparation comprising second antibodies that specifically bind the first antibodies;
iv) a third antibody preparation comprising third antibodies that specifically bind the second antibodies; and
wherein either the second antibodies or the third antibodies are conjugated to a detectable marker.

In an embodiment of the fourth aspect, the detectable marker may be an enzyme, for example, Horseradish Peroxidase (HRP) or alkaline phosphatase, as would be appreciated by a person of skill in the art. Appropriate substrates for HRP include chromogenic substrates (e.g., 3,3',5,5'-Tetramethylbenzidine (TMB), 3,3'-Diaminobenzidine (DAB), and 2,2'-azino-bis(3-ethylbenzthiazoline-6-sulphonic acid)) (ABTS) and chemiluminescent substrates (e.g., SuperSignala and ECL). A particularly preferred substrate is Alexa555 fluorophore labelled Tyramide.

In an alternative embodiment of the fourth aspect, the detectable marker may be a chemiluminescent moiety (e.g. an acridinium ester compound), a radioactive moiety (e.g. ³²P), or a fluorescent moiety (e.g. Fluorescein (FITC)). Other appropriate detectable labels and methods for their detection and their conjugation to antibodies will be well known to a person of skill in the art.

In an embodiment of any aspect of the invention, the solid support may be a microarray chip. Appropriate microarray chips may be constructed as follows: protein solutions (i.e. allergens) are initially prepared by diluting a stock solution of a protein to a final optimal concentration, in an optimal buffer (determined previously, see above). For each individual antigen, the final concentration may differ, as would be understood by a person of skill in the art. Protein solutions are then loaded into a 384-well plate. The plate and the solid support are then put inside a printer, for example a non-contact piezo-electric printer. The printer possesses a number of nozzles that draw the solutions from the wells and then dispenses them in drops onto the solid substrate (microarray). After dispensing each solution, the nozzles are then washed and made ready for the next solution. The printer has a camera, called a stroboscope, which monitors whether the solutions are properly dispensed by taking pictures of the drops being dispensed. If a solution is not dispensed properly, the stroboscope reports this. Any suitable optical support may be used to prepare the microarray. Generally, any glass support, or similar will be adequate. Various such supports will be well known to the skilled person.

Embodiments of the invention will now be described, by way of example only with reference to the Figures in which:
Figure 1 is a graphical representation of multiple allergen dose-response curves;
Figure 2A is a graphical representation of multiple binding-capacity curves; and
Figure 2B is a graphical representation of consolidated binding-capacity curves according to the invention.

### Example 1: Immunoassay with binding capacity calibration system.

Described is a calibration system suitable for precisely quantifying serum allergen-specific IgE, using a microarray-based immunoassay as a platform. The described immunoassay contains approximately 100 different allergenic extracts that cover a panel of approximately 100 different allergies.

The described calibration system can reliably describe the dose-response behaviour of all 100 allergen extracts. Each allergen extract is a unique compound with a different IgE binding capacity, i.e. different dose-response steepness. The present calibration system takes account of these different binding capacities to provide an accurate system for measuring allergen-specific IgE levels in a sample.

### Example microarray chip

The herein described system is a microarray-based test using miniaturized immunoassays designed for the measurement of up to approximately 103 allergens. Allergen extracts are immobilized onto chemically activated glass slides to generate the arrays. Each natural allergen extract is spotted onto the microarray in its optimal protein concentration and buffer (previously selected). Additionally, the microarray comprises positive controls (e.g. goat anti-mouse IgG) and negative controls (e.g. non-specific protein, such as bovine serum albumin), and capture anti-human IgE (polyclonal goat anti-human IgE) spotted in serial dilutions.

### Example antibody visualisation protocol

The following is an example of a protocol that may be used to visualise binding of IgE, either in a serum sample or a reference sample (the assays are carried out at the same time, with the same reagents where appropriate, such that potential environmental variations are controlled for), to the herein described microarray:
Separate arrays are first incubated with IgE samples (either serum or reference) and subsequently with monoclonal anti-human (or other appropriate antibody, depending on the assay samples) IgE antibody (for example, anti-human IgE mouse IgG), which will bind the human IgE from the serum or reference sample, if IgE is present and bound to the spotted allergens. Then a goat polyclonal anti-mouse IgG antibody conjugated with Horseradish peroxidase (HRP) is added to the array, followed by Alexa555 fluorophore labelled Tyramide. Appropriate washing steps are carried out between each antibody incubation step.

In the presence of hydrogen peroxide (H₂O₂), HRP enzyme converts Tyramide-Alexa555 into highly reactive, short-lived tyramide-Alexa555 radicals that react with nucleophilic residues in the vicinity of the HRP-target interaction site. This produces an emission of fluorescence at a specific wavelength (555 nm) of intensity proportional to the amount of bound HRP enzyme.

Use of the polyclonal antibody and of the HRP-Tyramide system (a non-liner signal amplification system) greatly increases the sensitivity of the microarray immunoassay test.

The above protocol provides a fluorescence intensity for each allergen spot that is plotted on a graph with serum sample concentration to provide a curve that is interpolated with a reference curve to quantify IgE level.

### Calibration method of invention

The herein described calibration method is exemplified by the following steps using the microarray chip of the invention:
1. Identification of dose-response curve for each allergen. A number of serum samples with known IgE reactivity are tested on the microarray chip. The signal intensity obtained from the allergens is collected and used to generate allergen dose-response curves.
2. Production of a panel of binding capacity curves. Serial dilutions of WHO/NIBSC International Reference IgE Preparation (from 0.1 to 100 International Unit/ml) are incubated onto the chips. IgEs are bound by the spotted capture-anti-human IgE and the signal intensity generated is measured and used to build a panel of binding capacity curves. Each curve corresponds to one of the different concentrations of capture anti-human IgE and is obtained by plotting the WHO/NIBSC IgE concentrations used for the incubation of the chip versus the corresponding obtained signal intensity. A number of binding curves is produced according to the number of different spots of capture anti-human IgE present on the chip (see Figure 2A, where each curve corresponds to one of the different concentrations of capture anti-human IgE spotted onto the arrays and was obtained by plotting the WHO/NIBSC IgE concentrations versus the correspondingly obtained signal intensity).
3. Clustering of binding capacity curves. Binding-capacity curves are then clustered in groups to represent different binding capacity (for example, Very high, High, Medium and Low binding capacity). A regression curve is produced for each group and stored in the software of the analyzer instrument (see Figure 2B, which shows binding capacity curves clustered into groups).
4. Allergens assigned binding capacity curve. According to the slope and shape of the allergen dose-response curves obtained as described in point 1, one of the binding-capacity curves (Master Curves) is assigned to each allergen.
5. Quantification of allergen-specific IgE. Using this calibration system, allergen-specific IgEs of an unknown patient serum are measured by interpolation of the signal intensity obtained from a particular allergen spotted onto the microarray chip to the specifically assigned binding capacity curve. Allergen reactivity is expressed in International Unit/ml and/or Class Score.
6. Using internal controls (adjuster) in each chip, the system can build the dose-response Internal Curve taking into account the storage and environment conditions of the slide adjusting the Master Curves obtained at point No. 4 accordingly. The internal calibration consists of running an algorithm to move the Master Calibration Curve based on the signal of the adjusters. For example, if the signal of the adjuster, whose expected value is 1000 units gives 950, the algorithm may lead to a shift in the Master Calibration Curve of 5%.

Unlike typical allergen immunoassays, which use a single calibration curve, the immunoassay system of the invention takes into account the differences in the binding capacity of each allergen. This provides a much more accurate assay for quantification of allergen-specific IgE in a sample.

## Claims

1. A method of quantifying multiple antigen-specific immunoglobulins in a test sample, the method comprising the steps of;
(i) assaying binding of a series of samples containing immunoglobulin of known antigen reactivity to multiple antigen components or fragments thereof immobilised on a first solid support,
(ii) comparing the level of binding in step (i) with the known reactivity to produce a dose response curve for each antigen component or fragment thereof,
(iii) assaying binding of a serial dilution of a reference immunoglobulin sample of the same immunoglobulin subtype as that used in part (i) with a known total amount of immunoglobulin to a serial dilution of anti-immunoglobulin antibodies, fragments or derivatives thereof, immobilised on the first, or a second solid support,
(iv) comparing the level of binding in step (iii) with the known total amount of reference immunoglobulin to produce a binding capacity curve for each anti-immunoglobulin antibody, fragment or derivative dilution,
(v) comparing the dose response curves produced in step (ii) with the binding capacity curves produced in step (iv), identifying the binding capacity curve that most closely matches the dose-response curve for each antigen or fragment thereof, and assigning the identified binding capacity curve as a binding capacity curve to each antigen or fragment thereof,
(vi) assaying binding of antigen-specific immunoglobulin in the test sample to the antigen components or fragments thereof immobilised on the first, second, or a third solid support, and
(vii) comparing the level of binding in step (vi), with respect to each individual antigen or fragment thereof, to the binding capacity curve assigned to that antigen or fragment thereof in step (v) and quantifying the level of antigen-specific immunoglobulin present in the test sample.

2. The method of Claim 1, wherein step (iv) further comprises clustering the binding capacity curves produced in step (iv) into representative binding capacity curves to represent different levels of binding capacity, and the representative binding capacity curves are used as the binding capacity curves produced in step (iv).

3. A method of calibrating a device suitable for assaying binding of multiple antigen-specific immunoglobulins to multiple antigens or fragments thereof immobilised on a solid support, the method comprising the steps of;
(i) assaying binding of a series of samples containing immunoglobulin of known antigen reactivity to multiple antigen components or fragments thereof immobilised on a first solid support,
(ii) comparing the level of binding in step (i) with the known reactivity to produce a dose response curve for each antigen component or fragment thereof,
(iii) assaying binding of a serial dilution of a reference immunoglobulin sample of the same subtype as that used in part (i) with a known total amount of immunoglobulin to a serial dilution of anti-immunoglobulin antibodies, fragments or derivatives thereof, immobilised on the first, or a second solid support,
(iv) comparing the level of binding in step (iii) with the known total amount of reference immunoglobulin to produce a binding capacity curve for each anti-immunoglobulin antibody, fragment or derivative dilution,
(v) comparing the dose response curves produced in step (ii) with the binding capacity curves produced in step (iv), identifying the binding capacity curve that most closely matches the dose-response curve for each antigen or fragment thereof, and assigning the identified binding capacity curve as a binding capacity curve to each antigen or fragment thereof, and
(vi) inputting the binding capacity curves generated in step (v) into the device such that the binding capacity curves for each antigen can be interpolated with signals produced from samples containing unknown amounts of immunoglobulin that specifically binds that antigen.

4. The method of Claim 3, wherein step (iv) further comprises clustering the binding capacity curves produced in step (iv) into representative binding capacity curves to represent different levels of binding capacity, and the representative binding capacity curves are used as the binding capacity curves produced in step (iv).

5. The method of claim 2 or claim 4, wherein the representative binding curves are clustered into very high binding, high binding, medium binding and low binding capacity curves.

6. The method of any of Claims 1 to 5, wherein the antigens are recombinant or derived from a natural extract, or a combination thereof, and optionally, wherein the antigens are purified.

7. The method of any of Claims 1 to 4, wherein the antigens are allergens, the immunoglobulin is IgE and the anti-immunoglobulin antibodies are anti-IgE antibodies.

8. Use of a kit of parts comprising:
a) a multi-allergen test system for carrying out any one of the methods of claims 1-7 comprising a serial dilution of anti-IgE antibodiesfragments or derivatives thereof immobilised on a first solid support, and allergen components or fragments thereof immobilised on the first solid support, or a second solid support, and positive and negative controls immobilised on the first, second or a third solid support; and
b) one or more of the following:
i) a reference IgE sample;
ii) a first antibody preparation comprising first antibodies that bind IgE;
iii) a second antibody preparation comprising second antibodies that specifically bind the first antibodies;
iv) a third antibody preparation comprising third antibodies that specifically bind the second antibodies; and
wherein either the second antibodies or the third antibodies are conjugated to a detectable marker.

9. The use of Claim 8, wherein the antigens are recombinant or derived from a natural extract, or a combination thereof, and optionally, wherein the antigens are purified.

10. The use of any of Claims 8 or 9, wherein the detectable marker is an enzyme, for example, Horseradish Peroxidase (HRP).

11. The use of any of Claims 8 or 9, wherein the detectable marker is a chemiluminescent moiety, a radioactive moiety, or a fluorescent moiety.

12. The method or Z fuse J Z of any of Claims 1 to 11, wherein the solid support is a microarray chip.

## Patentansprüche

1. Verfahren zum Quantifizieren mehrerer antigenspezifischer Immunglobuline in einer Testprobe, wobei das Verfahren die Schritte umfasst;
(i) Testen der Bindung einer Serie von Proben, die Immunglobulin bekannter Antigenreaktivität enthalten, an mehrere Antigenkomponenten oder Fragmente davon, die auf einem ersten festen Träger immobilisiert sind,
(ii) Vergleichen des Bindungsniveaus in Schritt (i) mit der bekannten Reaktivität, um eine Dosis-Effekt-Kurve für jede Antigenkomponente oder jedes Fragment davon zu erstellen,
(iii) Testen der Bindung einer Reihenverdünnung einer Referenz-Immunglobulinprobe des gleichen Immunglobulinuntertyps wie der in Teil (i) verwendete mit einer bekannten Gesamtmenge an Immunglobulin an eine Reihenverdünnung von Anti-Immunglobulin-Antikörpern, Fragmenten oder Derivaten davon, die auf dem ersten oder einem zweiten festen Träger immobilisiert sind,
(iv) Vergleichen des Bindungsniveaus in Schritt (iii) mit der bekannten Gesamtmenge an Referenz-Immunglobulin, um eine Bindungskapazitätskurve für jede/n/s Antiimmunoglobulin-Antikörper, Fragment oder Derivatverdünnung zu erstellen,
(v) Vergleichen der in Schritt (ii) erstellten Dosis-Effekt-Kurven mit den in Schritt (iv) erstellten Bindungskapazitätskurven, Identifizieren der Bindungskapazitätskurve, die der Dosis-Effekt-Kurve für jedes Antigen oder Fragment davon am ehesten entspricht, und Zuweisen der identifizierten Bindungskapazitätskurve als eine Bindungskapazitätskurve zu jedem Antigen oder Fragment davon,
(vi) Testen der Bindung von antigenspezifischem Immunglobulin in der Testprobe an die Antigenkomponenten oder Fragmente davon, die auf dem ersten, zweiten oder einem dritten festen Träger immobilisiert sind, und
(vii) Vergleichen des Bindungsniveaus in Schritt (vi) in Bezug auf jedes einzelne Antigen oder Fragment davon mit der Bindungskapazitätskurve, die diesem Antigen oder Fragment davon in Schritt (v) zugeordnet ist, und Quantifizieren des Niveaus des antigenspezifischen Immunglobulins, das in der Testprobe vorhanden ist.

2. Verfahren nach Anspruch 1, wobei Schritt (iv) ferner das Gruppieren der in Schritt (iv) erstellten Bindungskapazitätskurven in repräsentative Bindungskapazitätskurven umfasst, um unterschiedliche Niveaus der Bindungskapazität darzustellen, und die repräsentativen Bindungskapazitätskurven werden als die Bindungskapazitätskurven verwendet, die in Schritt (iv) erstellt wurden.

3. Verfahren des Kalibrierens einer Vorrichtung, die zum Testen der Bindung mehrerer antigenspezifischer Immunglobuline an mehrere Antigene oder Fragmente davon, die auf einem festen Träger immobilisiert sind, geeignet ist, wobei das Verfahren die Schritte umfasst;
(i) Testen der Bindung einer Serie von Proben, die Immunglobulin bekannter Antigenreaktivität enthalten, an mehrere Antigenkomponenten oder Fragmente davon, die auf einem ersten festen Träger immobilisiert sind,
(ii) Vergleichen des Bindungsniveaus in Schritt (i) mit der bekannten Reaktivität, um eine Dosis-Effekt-Kurve für jede Antigenkomponente oder jedes Fragment davon zu erstellen,
(iii) Testen der Bindung einer Reihenverdünnung einer Referenz-Immunglobulinprobe des gleichen Untertyps wie der in Teil (i) verwendete mit einer bekannten Gesamtmenge an Immunglobulin an eine Reihenverdünnung von Anti-Immunglobulin-Antikörpern, Fragmenten oder Derivaten davon, die auf dem ersten oder einem zweiten festen Träger immobilisiert wurden,
(iv) Vergleichen des Bindungsniveaus in Schritt (iii) mit der bekannten Gesamtmenge an Referenz-Immunglobulin, um eine Bindungskapazitätskurve für jede/n/s Antiimmunoglobulin-Antikörper, Fragment oder Derivatverdünnung zu erstellen,
(v) Vergleichen der in Schritt (ii) erstellten Dosis-Effekt-Kurven mit den in Schritt (iv) erstellten Bindungskapazitätskurven, Identifizieren der Bindungskapazitätskurve, die der Dosis-Effekt-Kurve für jedes Antigen oder Fragment davon am ehesten entspricht, und Zuweisen der identifizierten Bindungskapazitätskurve als eine Bindungskapazitätskurve zu jedem Antigen oder Fragment davon, und
(vi) Eingeben der in Schritt (v) erzeugten Bindungskapazitätskurven in die Vorrichtung, so dass die Bindungskapazitätskurven für jedes Antigen mit Signalen interpoliert werden können, die aus Proben erstellt wurden, die unbekannte Mengen an Immunglobulin enthalten, das dieses Antigen spezifisch bindet.

4. Verfahren nach Anspruch 3, wobei Schritt (iv) ferner das Gruppieren der in Schritt (iv) erstellten Bindungskapazitätskurven in repräsentative Bindungskapazitätskurven umfasst, um unterschiedliche Niveaus der Bindungskapazität darzustellen, und die repräsentativen Bindungskapazitätskurven werden als die Bindungskapazitätskurven verwendet, die in Schritt (iv) erstellt wurden.

5. Verfahren nach Anspruch 2 oder Anspruch 4, wobei die repräsentativen Bindungskurven in Kurven mit sehr hoher Bindungs-, hoher Bindungs-, mittlerer Bindungs- und niedriger Bindungskapazität gruppiert sind.

6. Verfahren nach einem der Ansprüche 1 bis 5, wobei die Antigene rekombinant sind oder von einem natürlichen Extrakt oder einer Kombination davon abgeleitet sind, und gegebenenfalls wobei die Antigene gereinigt sind.

7. Verfahren nach einem der Ansprüche 1 bis 4, wobei die Antigene Allergene sind, das Immunglobulin IgE ist und die Anti-Immunglobulin-Antikörper Anti-IgE-Antikörper sind.

8. Verwendung eines Teilesatzes, umfassend:
a) ein Multiallergen-Testsystem zum Durchführen eines der Verfahren nach Anspruch 1 bis 7, umfassend eine Reihenverdünnung von Anti-IgE-Antikörperfragmenten oder Derivaten davon, die auf einem ersten festen Träger immobilisiert sind, und Allergenkomponenten oder Fragmenten davon, die auf dem ersten festen Träger oder einen zweiten festen Träger immobilisiert sind, und positive und negative Kontrollen, die auf dem ersten, zweiten oder einem dritten festen Träger immobilisiert sind; und
b) eins oder mehrere der Nachfolgenden:
i) eine Referenz-IgE-Probe;
ii) eine erste Antikörperzubereitung, umfassend erste Antikörper, die IgE binden;
iii) eine zweite Antikörperzubereitung, umfassend zweite Antikörper, die spezifisch die ersten Antikörper binden;
iv) eine dritte Antikörperzubereitung, umfassend dritte Antikörper, die spezifisch die zweiten Antikörper binden; und
wobei entweder die zweiten Antikörper oder die dritten Antikörper an einen nachweisbaren Marker konjugiert sind.

9. Verwendung nach Anspruch 8, wobei die Antigene rekombinant sind oder von einem natürlichen Extrakt oder einer Kombination davon abgeleitet sind, und gegebenenfalls wobei die Antigene gereinigt sind.

10. Verwendung nach einem der Ansprüche 8 oder 9, wobei der nachweisbare Marker ein Enzym ist, beispielsweise Meerrettichperoxidase (HRP).

11. Verwendung nach einem der Ansprüche 8 oder 9, wobei der nachweisbare Marker eine chemilumineszierende Einheit, eine radioaktive Einheit oder eine fluoreszierende Einheit ist.

12. Verfahren oder Verwendung nach einem der Ansprüche 1 bis 11, wobei der feste Träger ein Microarray-Chip ist.

## Revendications

1. Procédé de quantification de multiples immunoglobulines spécifiques à des antigènes dans un échantillon test, le procédé comprenant les étapes de ;
(i) dosage de la liaison d'une série d'échantillons contenant une immunoglobuline de réactivité antigénique connue à de multiples composants antigéniques ou à des fragments de ceux-ci immobilisés sur un premier support solide,
(ii) comparaison du niveau de liaison à l'étape (i) à la réactivité connue pour produire une courbe dose-réponse pour chaque composant antigénique ou fragment de celui-ci,
(iii) dosage de la liaison d'une dilution en série d'un échantillon d'immunoglobuline de référence du même sous-type d'immunoglobuline que celui utilisé dans la partie (i) avec une quantité totale connue d'immunoglobuline à une dilution en série d'anticorps anti-immunoglobuline, de fragments ou dérivés de ceux-ci, immobilisés sur le premier ou un deuxième support solide,
(iv) comparaison du niveau de liaison à l'étape (iii) à la quantité totale connue d'immunoglobuline de référence pour produire une courbe de capacité de liaison pour chaque dilution d'anticorps anti-immunoglobuline, de fragment ou de dérivé,
(v) comparaison des courbes dose-réponse produites à l'étape (ii) aux courbes de capacité de liaison produites à l'étape (iv), identification de la courbe de capacité de liaison qui correspond le plus étroitement à la courbe dose-réponse pour chaque antigène ou fragment de celui-ci, et attribution de la courbe de capacité de liaison identifiée en tant que courbe de capacité de liaison à chaque antigène ou fragment de celui-ci,
(vi) dosage de la liaison d'une immunoglobuline spécifique à un antigène dans l'échantillon test aux composants antigéniques ou fragments de ceux-ci immobilisés sur le premier, le deuxième ou un troisième support solide, et
(vii) comparaison du niveau de liaison à l'étape (vi), par rapport à chaque antigène individuel ou fragment de celui-ci, à la courbe de capacité de liaison attribuée à cet antigène ou fragment de celui-ci à l'étape (v) et quantification du niveau d'immunoglobuline spécifique à un antigène présent dans l'échantillon test.

2. Procédé selon la revendication 1, ladite étape (iv) comprenant en outre le regroupement des courbes de capacité de liaison produites à l'étape (iv) dans des courbes de capacité de liaison représentatives pour représenter différents niveaux de capacité de liaison, et les courbes de capacité de liaison représentatives étant utilisées en tant que courbes de capacité de liaison produites à l'étape (iv).

3. Procédé d'étalonnage d'un dispositif approprié pour doser la liaison de multiples immunoglobulines spécifiques à des antigènes à de multiples antigènes ou fragments de ceux-ci immobilisés sur un support solide, le procédé comprenant les étapes de ;
(i) dosage de la liaison d'une série d'échantillons contenant une immunoglobuline de réactivité antigénique connue à de multiples composants antigéniques ou à des fragments de ceux-ci immobilisés sur un premier support solide,
(ii) comparaison du niveau de liaison à l'étape (i) à la réactivité connue pour produire une courbe dose-réponse pour chaque composant antigénique ou fragment de celui-ci,
(iii) dosage de la liaison d'une dilution en série d'un échantillon d'immunoglobuline de référence du même sous-type que celui utilisé dans la partie (i) avec une quantité totale connue d'immunoglobuline à une dilution en série d'anticorps anti-immunoglobuline, de fragments ou dérivés de ceux-ci, immobilisés sur le premier ou un deuxième support solide,
(iv) comparaison du niveau de liaison à l'étape (iii) à la quantité totale connue d'immunoglobuline de référence pour produire une courbe de capacité de liaison pour chaque dilution d'anticorps anti-immunoglobuline, de fragment ou de dérivé,
(v) comparaison des courbes dose-réponse produites à l'étape (ii) aux courbes de capacité de liaison produites à l'étape (iv), identification de la courbe de capacité de liaison qui correspond le plus étroitement à la courbe dose-réponse pour chaque antigène ou fragment de celui-ci, et attribution de la courbe de capacité de liaison identifiée en tant que courbe de capacité de liaison à chaque antigène ou fragment de celui-ci, et
(vi) entrée des courbes de capacité de liaison générées à l'étape (v) dans le dispositif de sorte que les courbes de capacité de liaison pour chaque antigène puissent être interpolées avec des signaux produits à partir d'échantillons contenant des quantités inconnues d'une immunoglobuline qui se lie spécifiquement à cet antigène.

4. Procédé selon la revendication 3, ladite étape (iv) comprenant en outre le regroupement des courbes de capacité de liaison produites à l'étape (iv) en courbes de capacité de liaison représentatives pour représenter différents niveaux de capacité de liaison, et les courbes de capacité de liaison représentatives étant utilisées en tant que courbes de capacité de liaison produites à l'étape (iv).

5. Procédé selon la revendication 2 ou la revendication 4, lesdites courbes de liaison représentatives étant regroupées en courbes de capacité de liaison très élevée, de capacité de liaison élevée, de capacité de liaison moyenne et de faible capacité de liaison.

6. Procédé selon l'une quelconque des revendications 1 à 5, lesdits antigènes étant recombinants ou dérivés d'un extrait naturel, ou une combinaison de ceux-ci, et éventuellement lesdits antigènes étant purifiés.

7. Procédé selon l'une quelconque des revendications 1 à 4, lesdits antigènes étant des allergènes, ladite immunoglobuline étant l'IgE et lesdits anticorps anti-immunoglobuline étant des anticorps anti-IgE.

8. Utilisation d'un kit de pièces comprenant :
a) un système de test multi-allergène permettant d'effectuer l'un quelconque des procédés selon les revendications 1 à 7 comprenant une dilution en série d'anticorps anti-IgEfragments ou de dérivés de ceux-ci immobilisés sur un premier support solide, et des composants allergènes ou des fragments de ceux-ci immobilisés sur le premier support solide ou un deuxième support solide, et des témoins positifs et négatifs immobilisés sur le premier, le deuxième ou un troisième support solide ; et
b) un ou plusieurs des éléments suivants :
i) un échantillon d'IgE de référence ;
ii) une première préparation d'anticorps comprenant des premiers anticorps qui se lient à l'IgE ;
iii) une deuxième préparation d'anticorps comprenant des seconds anticorps qui se lient spécifiquement aux premiers anticorps ;
iv) une troisième préparation d'anticorps comprenant des troisièmes anticorps qui se lient spécifiquement aux deuxièmes anticorps ; et
soit les deuxièmes anticorps soit les troisièmes anticorps étant conjugués à un marqueur détectable.

9. Utilisation selon la revendication 8, lesdits antigènes étant recombinants ou dérivés d'un extrait naturel, ou une combinaison de ceux-ci, et éventuellement lesdits antigènes étant purifiés.

10. Utilisation selon l'une quelconque des revendications 8 ou 9, ledit marqueur détectable étant une enzyme, par exemple, la peroxydase du raifort (HRP).

11. Utilisation selon l'une quelconque des revendications 8 ou 9, ledit marqueur détectable étant une fraction chimioluminescente, une fraction radioactive ou une fraction fluorescente.

12. Procédé ou utilisation selon l'une quelconque des revendications 1 à 11, ledit support solide étant une puce à microréseau.
